(19) Europäisches Patentamt
European Patent Office
Office européen des brevets

(11) EP 3 848 066 B1

(12) **EUROPEAN PATENT SPECIFICATION**

(45) Date of publication and mention of the grant of the patent:
**13.03.2024 Bulletin 2024/11**

(21) Application number: **19858375.9**

(22) Date of filing: **27.08.2019**

(51) International Patent Classification (IPC):
***A61L 31/04*** *(2006.01)* ***A61L 31/06*** *(2006.01)*
***A61L 31/14*** *(2006.01)*

(52) Cooperative Patent Classification (CPC):
**A61L 31/148; A61L 31/045;** A61L 2430/40

(86) International application number:
**PCT/JP2019/033549**

(87) International publication number:
**WO 2020/050102 (12.03.2020 Gazette 2020/11)**

(54) **ADHESION PREVENTION MATERIAL**
HAFTVERHINDERNDER STOFF
MATÉRIAU ANTI-ADHÉRENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.09.2018 JP 2018165810**

(43) Date of publication of application:
**14.07.2021 Bulletin 2021/28**

(73) Proprietor: **National Institute for Materials Science**
**Tsukuba-shi, Ibaraki 305-0047 (JP)**

(72) Inventor: **TAGUCHI Tetsushi**
**Tsukuba-shi, Ibaraki 305-0047 (JP)**

(74) Representative: **van Dam, Vincent**
**Octrooibureau Vriesendorp & Gaade B.V.**
**Koninginnegracht 19**
**2514 AB Den Haag (NL)**

(56) References cited:
**WO-A1-2017/126390** **WO-A1-2017/126390**
**WO-A1-2019/045081** **JP-A- 2011 025 013**
**JP-A- 2013 226 166** **JP-A- 2019 051 189**

- **ITO, TAICHI et al.: "Formation of a Biocompatible Film in vivo -from Peritoneal Adhesion to Drug Delivery System", MEMBRANE, vol. 36, no. 2, 2011, pages 63-70, XP009526848, ISSN: 0385-1036**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present invention relates to a material for use in preventing in a surgical site from adhering after surgery as defined in claim 1, specifically to a bioabsorbable adhesion prevention material comprising a gelatin derivative having a predetermined hydrophobic group as a main component as defined in claim 1.

### BACKGROUND OF THE INVENTION

An adhesion prevention material is defined as a bioabsorbable synthetic material applied directly to a surgical site during surgery to reduce adhesion after the surgery ("Specific Medical Treatment Materials and Their Material Prices (Material Price Reference)" 2016, Notification of the Ministry of Health, Labor and Welfare, No. 402), and is generally referred to as "absorbable adhesion prevention barrier." There are adhesion prevention materials in the form of sheet applied to a surgical site and those in the form of liquid or prepared into a liquid prior to use which are sprayed to a surgical site. Examples of the former include the one mainly composed of sodium hyaluronate and carboxymethyl cellulose (Seprafilm (trademark), Kaken Pharmaceutical Co., Ltd.), the one mainly composed of gelatin (Gelfilm (trademark), Pfizer Japan Inc., and Patent Document 1), and examples of the latter include the one consisting of N-hydroxysuccinimide dextrin and sodium carbonate/sodium hydrogen carbonate (Adspray (trademark),Terumo Corporation).

The adhesion prevention material is required to cover and protect the tissue surface. However, an adhesion prevention material in the form of film is difficult to be applied conformingly to a complicated shape of a surgical site. Seprafilm, once it gets wet with water, shows significantly degraded handling property to make it practically impossible to adjust attached site thereof. In this regard, the spray type materials can be applied more widely and conformingly to a surgical site.

Meanwhile, the inventors of the present invention have been developing surgical sealants administered to the tissue by spraying a gelatin derivative with hydrophobic groups introduced to the gelatin (hereinafter may be referred to as "hydrophobically modified gelatin") and a curing agent (for example, Patent Document 2 and Patent Document 3). The sealants having the hydrophobic group feature a high affinity to the tissue and have a significantly high sealing strength compared with that of gelatin without hydrophobic groups and that of a fibrin sealant.

Patent Document 1: JP2013-226166A
Patent Document 2: WO2014/112208
Patent Document 3: Japanese Patent No. 5995128

### SUMMARY OF THE INVENTION

### PROBLEMS TO BE SOLVED BY THE INVENTION

It was concerned that the aforesaid sealants undesirably adhere to tissues besides those of a surgical site due to its high adhesiveness to the tissues. However, surprisingly, it has been found that the sealants after being cured to a film shows an excellent adhesion prevention effect, thereby completing the present invention.

### MEANS TO SOLVE THE PROBLEMS

Thus, the present invention is as follows.

Material for use in preventing a surgical site from adhering after surgery, said material consisting of:

(1) a first agent comprising a gelatin derivative, the gelatin derivative

(a) including a structure represented by the following formula,

$$\text{GltnNH-CHR}^1\text{R}^2,$$

in the formula, Gltn represents a gelatin residue, $R^1$ represents an alkyl group with 5 to 17 carbon atoms, and $R^2$ represents a hydrogen atom or an alkyl group with 5 to 17 carbon atoms;

(b) having imino group/ amino group (molar ratio) of 1/99 to 30/70; and

(c) having a weight average molecular weight of 10,000 to 50,000; and

(2) a second agent comprising a crosslinker for the gelatin derivative.

## EFFECTS OF THE INVENTION

Because the adhesion prevention material can be administered to the tissue by spraying, it can be easily applied even to a surgical site having a complicated shape. A cured film of the adhesion prevention material exhibits an excellent adhesion prevention property. It adheres strongly to a surgical site, and therefore can be used as an adhesive material for adhesion prevention or as a surgical sealant having adhesion prevention property. Moreover, the main component of the gelatin derivative can be produced in an aqueous solvent, and thus it is safe for manufacturing environment and for the body. Further, it can be synthesized simply by one step process with a high yield.

## BRIEF DESCRIPTION OF THE DRAWINGS

Fig. 1 is a graph showing the number of adhered cells.
Fig. 2 is a schematic diagram of a device used for a cell permeability test.
Fig. 3 is a graph showing cell permeability.
Fig. 4a shows photographs of cross sections of tissues at a surgical site one week after the surgery.
Fig. 4b shows photographs of cross sections of tissues at the surgical site two weeks after the surgery.
Fig. 5 is a graph showing results of burst strength.
Fig. 6 shows photographs of cross sections of specimens after burst strength measurement.
Fig. 7 is a schematic diagram of a device used for a protein permeability test.
Fig. 8 is a graph showing protein permeability.
Fig. 9 is a graph showing a change in degree of swelling with time.
Fig. 10 is a graph showing a weight reduction according to the enzymatic decomposition.

## DESCRIPTION OF PREFERRED EMBODIMENTS

An adhesion prevention material for use as defined in the appended claims is of two-component type consisting of a first agent comprising a gelatin derivative as a main component that substantively forms a cured film and a second agent comprising a crosslinker for the gelatin derivative. The first agent and the second agent are separately packed to be provided and mixed for use. Details of these agents will be described below.

<First Agent>

The first agent in the adhesion prevention material for use as defined in the appended claims comprises the gelatin derivative. The gelatin derivative has hydrophobic groups bonded via an imino group, that is, -NH-, and has a structure represented by the following formula.

$$\mathrm{GltnNH{-}CHR^1R^2}$$

In the formula, Gltn represents a gelatin residue, $R^1$ represents an alkyl group with 5 to 17 carbon atoms, and $R^2$ represents a hydrogen atom or an alkyl group with 5 to 17 carbon atoms. N is mainly derived from an ε-amino group of lysine (Lys) in the gelatin. Preferably, $R^2$ is a hydrogen atom. The NH structure in this formula (1) can be detected by, for example, a band around 3300 $cm^{-1}$ in FT-IR spectrum.

When $R^2$ is the alkyl group with 5 to 17 carbon atoms, it may the same as or different from $R^1$. The alkyl group may be branched. Examples of the alkyl group include a hexyl group, an octyl group (or a caprylic group), a nonyl group (or a pelargonyl group), a dodecyl group (or a lauryl group), a tetradecyl group (or a myristyl group), and the like. Preferably, $R^1$ is a linear alkyl group with carbon atoms 5 to 11, and $R^2$ is a hydrogen atom.

A ratio of derivatization in the gelatin derivative, in terms of mol% of the imino group to which the alkyl group is bonded relative to an amount of the amino group in the original gelatin, ranges from 1 to 20 mol%, and preferably from 5 to 10 mol%. In other words, imino group/amino group (molar ratio) in the obtained gelatin derivative ranges from 1/99 to 20/80, and preferably from 5/95 to 10/90. The ratio of derivatization can be determined by quantitating the amino group in the original gelatin and the amino group after the alkyl group has been bonded by a 2,4,6-trinitrobenezene sulfonic acid method (TNBS method) or by identifying and quantitating the alkyl group by NMR or the like.

The original gelatin may be any one of natural origin gelatin, synthesized gelatin, fermented gelatin, and genetically engineered gelatin, and preferably a gelatin derived from an animal such as porcine or bovine gelatin, or a gelatin derived from fish, such as Alaska Pollock, is used. The gelatin may be any one of an acid-treated gelatin, an alkaline-treated gelatin, and a genetically engineered gelatin, among which the alkaline-treated gelatin is preferred and a low endotoxin gelatin is more preferred. Regarding a range of molecular weight of the gelatin, a range in which a weight average molecular weight (Mw) of the gelatin derivative becomes 10,000 to 100,000 is preferred, and a range in which it becomes 10,000 to 50,000 is more preferred. The molecular weight can be measured by gel permeation chromatography (GPC) in accordance with a conventional method.

The first agent may contain an underivatized gelatin, in addition to the aforesaid gelatin derivative. As such gelatin, aforesaid various gelatins can be used. An amount of the underivatized gelatin ranges from 0 to 99 wt%, preferably from 0 to 50 wt% relative to a total weight of the gelatin derivative and the underivatized gelatin.

The first agent may further contain an aqueous solvent to dissolve or disperse the gelatin derivative. From the handling viewpoint, the gelatin derivative may be dissolved or dispersed in the aqueous solvent to be provided as an aqueous liquid (hereinafter may be simply referred to as "aqueous solution"). As the aqueous solvent, ultrapure water, saline, a buffer solution containing various acids, such as boric acid, phosphoric acid, or carbonic acid, and a salt thereof, or a mixture of these solvents can be used. Preferably, a boric acid buffer having a pH of from 8 to 11, and more preferably from 9 to 10 is used. This aqueous solvent is used in such an amount that a concentration of the gelatin derivative ranges from 10 to 80 wt/v%, and preferably from 15 to 30 wt/v%. When the underivatized gelatin is contained, a total concentration of the gelatin derivative and the underivatized gelatin falls in the aforesaid concentration.

<Second Agent>

In the present invention, the second agent is a crosslinker for the gelatin derivative which crosslinks to from a structure such as a film in soluble in water or body fluids such as blood. As the crosslinker, at least one kind of a crosslinker is used that has at least two functional groups per molecule reactive with amino groups in the gelatin, mainly the primary amino groups at side chains. Examples of the crosslinker include genipin, polybasic acid activated with N-hydroxysuccinimide or N-hydroxysulfosuccinimide, aldehyde compounds, acid anhydrides, and diisothiocyanate.

Examples of the polybasic acid include tartaric acid, citric acid, malic acid, glutaric acid, glutamic acid, aspartic acid, oxaloacetic acid, cis-aconitic acid, 2-ketoglutaric acid, polytartaric acid, polycitric acid, polymalic acid, polyglutamic acid, polyaspartic acid, carboxymethylated dextrin, carboxymethylated dextran, carboxymethylated starch, carboxymethylated cellulose, carboxymethylated chitosan, carboxymethylated pullulan, and active-esterified derivatives thereof, for example, disuccinimidyl glutarate (DSG), disuccinimidyl suberate (DSS), disuccinimidyl tartrate (DST).

Other examples include polybasic acid esters of polyethylene glycol or polyethylene glycol ether in which at least one carboxyl group that is not reacted with polyethylene glycol is active-esterified, for example, 4,7,10,13,16-pentaoxanonadecanedioic acid di(N-succinimidyl), and polyethylene glycol di(succinimidyl succinate) (SS-PEG-SS) represented by the following formula:

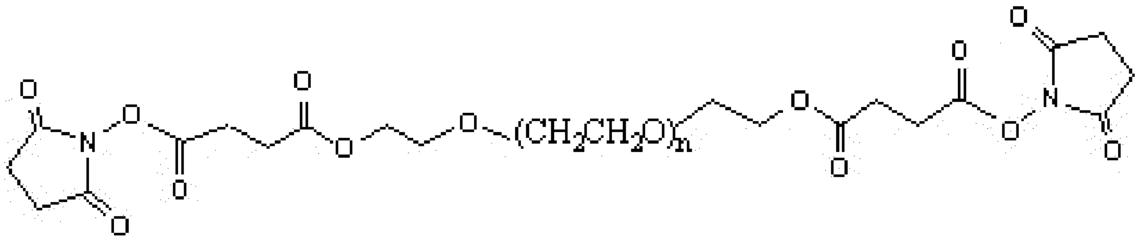

(n represents such a number that Mw ranges from about 10,000 to 20,000) and, pentaerythritol polyethylene glycol ether tetrasuccinimidyl glutarate (4S-PEG) represented by the following formula:

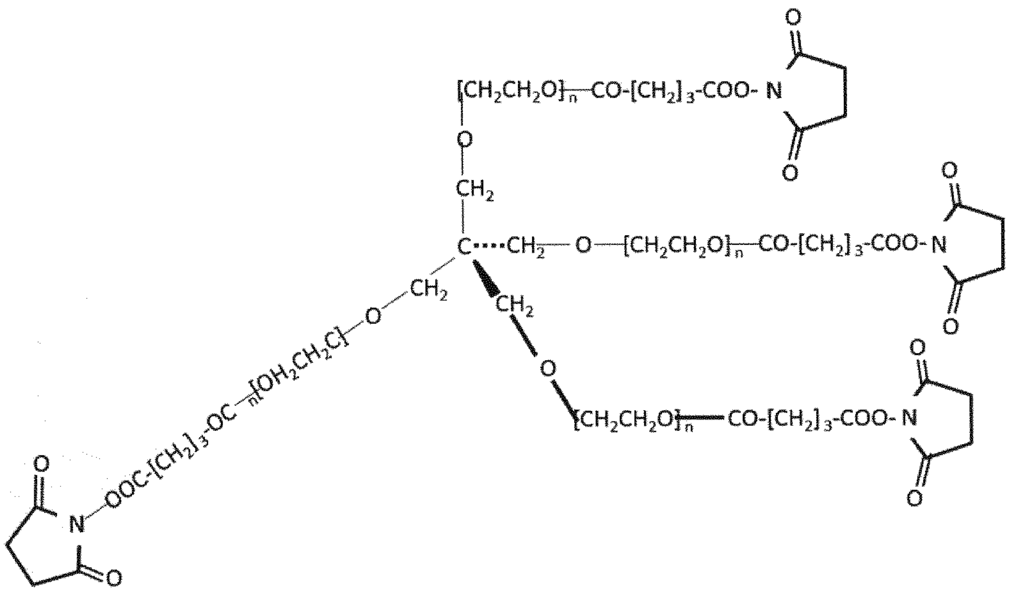

(n is such a number that Mw ranges from about 3,000 to 30,000, preferably about 5,000 to 27,000, and more preferably about 10,000 to 20,000.)

Examples of the aldehyde compound include aldehyde group-introduced polysaccharide in which two or more aldehyde groups are introduced per molecule, such as aldehyde group-introduced starch, aldehyde group-introduced dextran, aldehyde group-introduced dextrin, and aldehyde group-introduced hyaluronic acid. Examples of the acid anhydride include glutaric acid anhydride, maleic acid anhydride, and succinic acid anhydride. Example of the diisothiocyanate includes hexamethylenediisothiocyanate or the like. Among these, aforesaid activated polybasic acid ester of polyethylene glycol and the aldehyde group-introduced polysaccharide are preferably used.

The crosslinker is used in such an amount that a functional group in the crosslinker, for example, an ester group activated with N-hydroxysuccinimide, per mole of the amino group of the gelatin derivative ranges from 0.2 to 3 equivalents, preferably 0.2 to 2 equivalents, and more preferably 0.2 to 1.0 equivalent, and most preferably from 0.2 to 0.6. A mixture of two or more kinds of the crosslinkers may be used in such an amount that a total equivalent amount thereof falls in the above range.

The second agent may further contain an aqueous solvent to dissolve the crosslinker. However, it is preferred that the crosslinker and the aqueous solvent are provided in different containers, and at most about two hours before the use, appropriate amounts thereof are mixed into an aqueous solution (hereinafter may be simply referred to as "aqueous solution") to be used. The above-described aqueous solvents for the first agent are usable as the aqueous solvent. Preferably, a phosphate buffer having a pH of from 3 to 8, and more preferably from 4 to 6 is used. Most preferably, ionic strengths of aqueous solvents of the both agents are adjusted so that a mixture of the same volume of the first agent solution and the second agent solution has a pH of from 8 to 9. For example, an aqueous solution of the first agent in a borate buffer having a pH of 9 and an ionic strength of 0.05 to 0.1, and an aqueous solution of the second agent in a phosphate buffer having a pH of 4 and an ionic strength of from 0.01 to 0.03 can make an equivolume mixed solution having a pH of from 8 to 9. Another example is a combination of an aqueous solution of the first agent in a borate buffer having a pH of 10 and an ionic strength of 0.05 to 0.1, and an aqueous solution of the second agent in a phosphate buffer having a pH of 4 and an ionic strength of from 0.01 to 0.07.

A concentration of crosslinker in the second agent is adjusted such that an equivalent of the functional group in the second agent relative to an equivalent of the amino group in the first agent, that is, (the functional group equivalent in the second agent/the amino group equivalent in the first agent) falls in the above-described range. A mixture of two or more kinds of crosslinkers may be used in such an amount that a total of equivalents thereof falls in the above-described range.

<Additive>

Various additives may be further contained in the first agent and/or the second agent in such an amount that they do not interfere with the object of the present invention. Examples of the additive include a colorant, a pH adjuster, a viscosity modifier, and a preservative. Preferably, a colorant, for example, brilliant blue is added in an aqueous solution of the first agent or the second agent to highlight an applied site of the adhesion prevention material. The additive may be added in an amount of, for example, from 10 to 100 μg/mL.

<Manufacturing Method>

The adhesion prevention material for use as defined in the appended claims can be obtained by preparing and packing the first agent and the second agent, and sterilizing as desired.

[Preparation Method of First Agent]

(1) Preparation of an aqueous solution of an original gelatin

A starting original gelatin is dissolved in an aqueous solvent at a concentration of from 5 to 50 wt/v% by heating at 40 to 90°C. A mixture of water and a water-soluble organic solvent is used as the aqueous solvent. As the water-soluble organic solvent, alcohol with 1 to 3 carbon atoms, ester having 1 to 3 carbon atoms, or the like can be used, and preferably ethanol is used.

(2) Derivatization

A derivatizing agent having an alkyl group to be introduced is added to the aqueous solution of the gelatin obtained in the step (1) and the mixture thus obtained is subjected to a reaction while stirring for a predetermined period of time. As the derivatizing agent, an aldehyde or a ketone having the alkyl group, for example, dodecanal, tetradecanal, and decylethyl ketone, is used. A reaction temperature ranges from 30 to 80°C, a reaction time ranges from 0.5 to 12 hours, and usually, mere stirring can produce a gelatin in which the alkyl group is bonded to the amino group of the gelatin in the form of Schiff base (GltnN = $CR^1R^2$). The aldehyde is used in an amount of from one to four times, more preferably from one to two times, the stoichiometric amount corresponding to a desired ratio of derivatization.

Next, this Schiff base is reduced. As a reductant, a known reducing agent can be used such as sodium cyanoborohydride ($NaBH_3CN$), sodium triacetoxyborohydride ($NaBH(OAc)_3$), 2-picoline borane, and pyridine borane, among which 2-picoline borane is preferred. The picoline borane is stable to allow a reductive amination reaction of the aldehyde or the ketone in an aqueous solvent in one step (one-pot). Additionally, a yield of 80 to 90% can be achieved, which is significantly higher than 70 to 75% achieved by sodium cyanoborohydride. Preferably, 2-picoline borane is used in an amount of from 1 to 3 equivalents relative to the equivalent of the derivatizing agent.

(3) Purification

To the reaction solution obtained in the step (2), a large excess amount of a poor solvent, for example, cold ethanol is added to precipitate the gelatin derivative. After being filtered out, the precipitate is cleaned with ethanol or the like to obtain a final product.

(4) Preparation of first agent

The gelatin derivative obtained in the step (3) is provided in the form of powder or aqueous solution in an aqueous solvent such as a boric acid buffer and is packed in a container. As the container, a vial, a bottle, a dispenser, and a syringe made of glass or plastic can be used. If desired, the original gelatin and other additives may be added. When the gelatin derivative is provided in the form of powder, an aqueous solvent such as a boric acid buffer is provided in a separate container. When the gelatin derivative is provided in the form of aqueous solution, it may be placed in one of the syringes of a dual syringe dispenser or the like that can mix both agents at a tip thereof which is used when the adhesion prevention material is applied to an affected site. Needless to say, the adhesion prevention material for use as defined in the appended claims may include a double syringe dispenser, a vial for mixing, a spare aqueous solvent, or the like as accessories.

[Preparation Method of Second Agent]

The crosslinkers exemplified above as the second agent may be synthesized by known methods, or commercially available crosslinkers may be used. The crosslinker and an aqueous solvent to dissolve the crosslinker such as a phosphate buffer are provided in separate containers, for example, the crosslinker in a glass vial and the aqueous solvent in a plastic bottle. Additives may be added as desired. The first agent and the second agent are provided in such amounts that a ratio, (an equivalent of functional group of the crosslinker/an equivalent of amino group of the gelatin derivative), ranges from 0.2 to 2. However, the first agent and the second agent may be provided in such a manner that the first agent and second agent can be independently supplemented.

<Sterilization>

Next, the first agent in the form of aqueous solution packed in a dispenser etc. or in the form of a combination of the gelatin derivative powder packed in a vial etc. and an aqueous solvent packed in a bottle etc., and the second agent in the form of a combination of a crosslinker in the form of powder packed in a vial etc. and an aqueous solvent to dissolve this crosslinker packed in a bottle etc. are each sterilized. A preferred method of sterilizing the gelatin derivative powder and the crosslinker in the form of powder is radiation sterilization. Examples of the radiation include electron beam, gamma ray, and bremsstrahlung, among which electron beam sterilization is preferred. A total absorbed dose may be at least 20 kGy, which is widely used (The Japanese Pharmacopoeia 14th edition, Part II, reference information, page 1235, the right column, 2.2 Radiation Method), and preferably ranges from 25 kGy to 45 kGy. As far as a total absorbed dose is at least 20 kGy, irradiation of electron beam may be divided into a plurality of times to avoid damages to the gelatin derivative or the crosslinker while maintaining the sterilization effect. For example, in place of sterilization at a total absorbed dose of 30 kGy, irradiation at 10 kGy may be repeated three times. A method of sterilizing an aqueous solution of the gelatin derivative is preferably autoclaving or filter sterilization.

<Application Method to Tissues or the Like>

Also disclosed is a method of preparing an adhesion prevention material film comprising the step of administering the first agent and the second agent to an affected site of a human or non-human subject, and a method for preventing an adhesion comprising the method of preparing the adhesion prevention material film. The adhesion prevention material for use as defined in the appended claims is applicable to a ruptured site of the skin, blood vessels, tendons, nerves, the intestine, and tubular tissues such as the lymphatic vessel, and organs such as the liver, the pancreas, and the heart. Especially, the adhesion prevention material is preferably applied to, for example, wet tissues such as blood vessels and the lung. As the application method of the adhesion prevention material, the second agent is dissolved into an aqueous solution preferably immediately before the use as described above. The concentration of the crosslinker is as described above. The obtained aqueous solution of the second agent may be placed in an empty syringe of a double syringe dispenser, of which other syringe is filled with an aqueous solution of the first agent, and the first and the second agent are applied or sprayed with an air- assisted spray to an affected site of a subject. After the administration, a film is formed in several minutes to 10 minutes, and thus the affected site can be sealed.

## EXAMPLES

While the present invention will be described with reference to the Examples, the present invention is not limited to these examples.

<Preparation of Gelatin Derivative>

A gelatin derivative was prepared by the method described in Patent Document 3 (Japanese Patent No. 5995128). The method of preparing the gelatin derivative 1 using octanal will be described as an example.

Gelatin derived from Alaska Pollock (Mw 33,000, manufactured by Nitta Gelatin Inc.) in an amount of 30 g was dissolved in 105 mL of water at 50°C, to which a solution produced by dissolving 139 $\mu$L of n-octanal, which is a stoichiometric amount corresponding to 10 mol% of amino group in this gelatin, in 40 mL of ethanol was added and stirred for one hour at 50°C, followed by adding a solution obtained by dissolving 143 $\mu$L of 2-picoline borane in 5 mL of ethanol. After the reaction mixture thus obtained was stirred for 17 hours at 50°C, the reaction mixture was dropped in cold ethanol in an amount 10 times volume of the reaction mixture to precipitate the gelatin derivative, and suction filtration was performed. After being cleaned three times with the cold ethanol, the obtained precipitate was filtered off. The filtration residue thus obtained was dried under reduced pressure to obtain the gelatin derivative 1.

An infrared spectrum (FTIR-8400, Shimadzu Corporation) and a $^{13}$C-NMR spectrum (AL300, JEOL Ltd.) of the gelatin derivative 1 were taken, thereby the derivatization was confirmed. In the infrared spectrum, enhanced peaks of a secondary amine at 3280cm$^{-1}$ and a methylene group at 2936cm$^{-1}$ and 2879cm$^{-1}$, and in the $^{13}$C-NMR spectrum, a peak assigned to a primary carbon at 17 to 17.5 ppm were respectively observed, and thus the introduction of the alkyl group was confirmed.

A ratio of the derivatization was measured by the following method. The gelatin derivative 1 was dissolved in a dimethylformamide/water-mixed solvent with a volume ratio 1:1 to obtain a 0.1 w/v% solution. In this solution, 0.1 v/v% triethylamine aqueous solution and 0.1 w/v% trinitrobenzenesulfonic acid aqueous solution were added and stirred for about one minute to react trinitrobenzenesulfonic acid with amino groups in the gelatin derivative. Subsequently, light absorbance at 340 nm was measured with a microplate reader (SPARK 10M, Tecan Japan Co., Ltd.). The ratio of derivatization of the gelatin derivative 1 was determined to be 7.2 mol%.

Except that n-decanal or n-dodecanal in place of n-octanal and a gelatin of Mw 38,000 were used, gelatin derivatives shown in Table 1 were prepared in the same manner as the above-described preparation method of the gelatin derivative. Note that, in Table 1 and so on, the expression "C8", for example, represents a gelatin derivatized with C8 aldehyde ($R^1$ is a heptyl group and $R^2$ is a hydrogen atom in Formula (1)).

[Table 1]

| Gelatin derivative | Name | Gelatin molecular weight (Mw) | Ratio of derivatization (mole%) |
|---|---|---|---|
| 1 | C8-1 | 33,000 | 7.2 |
| 2 | C8-2 | 38,000 | 8 |
| 3 | C10 | 33,000 | 6.5 |
| 4 | C12 | 33,000 | 5.5 |

The gelatin derivatives were each dissolved at a concentration of 15 w/v% in a 0.1 M boric acid buffer with pH 9.5 , , thus preparing the first agent.

<Preparation of Second Agent>

The pentaerythritol polyethylene glycol ether tetrasuccinimidyl glutarate (4S-PEG, manufactured by NOF CORPORATION) was used as the second agent. Immediately before characterizations described below, 4S-PEG was dissolved in 0.01 M phosphate buffer with pH 4.0 in such an amount that an equivalent of 4S-PEG was 0.4 (0.5 equivalent in Example 4) per equivalent of residual amino group of the first agent when mixed with the same volume of the first agent.

<Preparation Method of Film>

The first agent and the second agent in the same volume were injected on a base material in each test with a double syringe dispenser to apply a film of an Example on the tissues. As shown in Table 2, as Comparative Examples, gelatin before derivatization (Comparative Example 1: Org), fibrin (Comparative Example 2: BOLHEAL, product name, manufactured by Chemo-Sero-Therapeutic Research Institute), Seprafilm (Comparative Example 3: HA/CMC Film; trademark, Kaken Pharmaceutical Co., Ltd.) were used in accordance with respective package insert. Comparative Examples 2 and 3 were used in such an amount to have an identical area to those of Examples.

[Table 2]

| | Name | Crosslinker equivalent |
|---|---|---|
| Example 1 | C8-1 | 0.4 |
| Example 2 | C8-2 | 0.4 |
| Example 3 | C10_0.4 | 0.4 |
| Example 4 | C10_0.5 | 0.5 |
| Example 5 | C12 | 0.4 |
| Comparative Example 1 | Org | 0.4 |
| Comparative Example 2 | Fibrin | - |
| Comparative Example 3 | Seprafilm | - |

Note that, in Fig. 3 and so on, Comparative Example 2 is referred to as "Fibrin" Comparative Example 3 as "HA/CMC."

To evaluate adhesion prevention property, an in vitro cell adhesion test, an in vitro cell permeation test, and an in vivo adhesion prevention were conducted.

<Cellular Adhesiveness>

On bottom surfaces of 48-well plate, each of the first agents and the second agents of Examples 2 to 5 and

Comparative Example 1 were administered in an amount of 150 μL to form a film, which was irradiated with ultraviolet rays for one hour for sterilization. L929 fibroblast cells were seeded on the film (1.0 × $10^5$ cells/well) and cultured under carbon dioxide gas in a RPMI-1640 culture medium containing 10% fetal bovine serum and 1% solution of penicillin/streptomycin at 37°C. After 24 hours, the cells were cleaned with D-PBS and the number of cells attached on the film was counted using the WST-8 assay kit. A well without a film was used as a control.

Fig. 1 shows the results. As seen from the figure, all Examples exhibited less adhered cells compared with the control, indicating excellent adhesion prevention property in vitro.

<Cell Permeability>

Cell permeability was evaluated using a cell culture insert (Transwell insert, a PTFE membrane, pore size 8.0 μm, Corning Incorporated) as schematically illustrated in Fig. 2. On bottom surfaces of the insert, each of the first agents and the second agents of Example 1 and Comparative Example 1 in an amount of 100 μL and Comparative Examples 2 and 3 in an amount to make a film with the same surface area were administered to form a film, which was irradiated with ultraviolet rays for one hour for sterilization. L929 fibroblast cells were seeded on the film (1.0 × $10^6$ cells/well) and cultured under carbon dioxide gas in a RPMI-1640 culture medium containing 10% fetal bovine serum and 1% solution of penicillin/streptomycin at 37°C. After 24 hours, the number of cells permeated to the lower portions of the well was counted and a ratio (%) to the non-treated well, in which a film was not formed, was obtained. Fig. 3 shows the results.

As seen from Fig. 3, the cell permeability is significantly lower in Example compared with that of Seprafilm, which is considered to contribute to the adhesion prevention property.

<In Vivo Adhesion Prevention Property Evaluation>

Celiotomy was performed on a rat (Wistar, a 7-weeks female, CHARLES RIVER LABORATORIES JAPAN, INC.) to prepare a cecal abdominal wall defect model. The cecum was scrubbed with a medical gauze to bleed, thus damaging the cecal wall. By excising a predetermined size (1.0 × 2.0 cm) of the abdominal wall opposed to the damaged part with a medical knife, the abdominal wall defect was prepared. After application of each adhesion prevention material of Example 1 and Comparative Examples 1 to 3 to both of the abdominal wall defect and the cecum damage so as to cover the entire area of the damage, the incised site was sutured.

After one week and two weeks of the surgery, the rat was sacrificed, specimens of cross sections at three locations of the tissues of the surgical site were taken, fixed in a 10% formalin neutral buffer, stained with hematoxylin-eosin s, and observed with an optical microscope. Fig. 4a shows photographs after one week, and Fig. 4b shows photographs after two weeks. In the figures, "Untreated" means nothing was applied to the defect site. Tables 4 and 5 show the results of evaluations of a degree of adhesion of each specimen according to the criteria shown in Table 3. The larger the number of specimens having a low score is, the lower the degree of adhesion was.

[Table 3]

| Score | Abdominal adhesion state |
|---|---|
| **0** | No adhesion |
| **1** | Thin film-shaped adhesion |
| **2** | Thin adhesion at a plurality of sites |
| **3** | Thick adhesion at one site |
| **4** | Thick adhesion due to adhesion of sole portion or thick adhesion at a plurality of sites |
| **5** | Thick adhesion involving blood vessel or thick adhesion due to adhesion of sole portion at a plurality of sites |

[Table 4]

| Adhesion score | After one week (n = 3) | | | | |
|---|---|---|---|---|---|
| | No treatment | Org | C8-1 | Fibrin | Seprafilm |
| 5 | 0 | 0 | 0 | 0 | 0 |
| 4 | 3 | 0 | 0 | 0 | 0 |
| 3 | 0 | 0 | 0 | 2 | 0 |

(continued)

| Adhesion score | After one week (n = 3) | | | | |
|---|---|---|---|---|---|
| | No treatment | Org | C8-1 | Fibrin | Seprafilm |
| 2 | 0 | 0 | 0 | 0 | 0 |
| 1 | 0 | 0 | 0 | 1 | 0 |
| 0 | 0 | 3 | 3 | 0 | 3 |

[Table 5]

| Adhesion score | After two weeks (n = 3) | | | | |
|---|---|---|---|---|---|
| | No treatment | Org | C8-1 | Fibrin | Seprafilm |
| 5 | 0 | 0 | 0 | 0 | 0 |
| 4 | 3 | 0 | 0 | 0 | 0 |
| 3 | 0 | 0 | 0 | 1 | 0 |
| 2 | 0 | 0 | 0 | 1 | 0 |
| 1 | 0 | 0 | 0 | 1 | 1 |
| 0 | 0 | 3 | 3 | 0 | 2 |

As shown in Tables 4 and 5, while significant adhesion was observed in the specimen that was not treated and the tissue to which the fibrin was applied, adhesion did not occur at all even after two weeks in Example 1 whereby an excellent adhesion prevention property was confirmed.

<Sealing Strength>

In accordance with ASTM (F2392-04), using porcine large intestine (φ30 mm) as the base material, a sealing strength (pressure-resistance) was measured. The first agent and the second agent of each of Example 1 and Comparative Examples 1 to 3 were mixed each in an amount of 100 μL (equivalent of succinimide ester group in the crosslinker/equivalent of amino group in the gelatin derivative = 0.4), which was applied to the porcine large intestine to form a 1.0 mm-thick film having a diameter of 15mm of the adhesion prevention material. After the application, a pressure bonding was performed for 10 minutes with a load of 5.0 g/mm$^2$ followed by flowing physiological saline at 37°C was flowed at 2 mL/minute, and then a pressure at burst was measured. Fig. 5 shows the results. Further, the specimen tissue after the burst was fixed using a neutral buffered formalin solution followed by staining with hematoxylin-eosin, and then an interface between the tissue and the sample was observed (Fig. 6).

As illustrated in Fig. 5, the adhesion prevention material for use as defined in the appended claims exhibited significantly high sealing strength compared with those of the other samples, which reconfirmed the results described in Patent Document 3. As shown in Fig. 6, while the fibrin film and the Seprafilm were peeled off at the interface between the film and the porcine large intestine, a cohesive failure occurred in the film itself of the adhesion prevention material of the present invention. This result shows that the adhesion prevention material for use as defined in the appended claims is excellent also in adhesive strength.

<Protein Permeability>

Using a cell culture insert (Transwell insert, PTFE membrane, pore size: 8.0 μm, Corning Incorporated) as illustrated in Fig. 7, the protein permeability was studied in accordance with the method described in D. Nwa et al., J. Biomed. Mater. Res. B, 2013, 101B, 1251-1258. On bottom surfaces of the insert, the first agent and the second agent were each administered in an amount of 100 μL to form films, which were irradiated with ultraviolet rays for one hour for sterilization. The insert was dipped in 0.9 mL of D-PBS. On the film, 0.1 mL of a 0.1 w/v% aqueous solution of fluorescein isocyanate-labeled albumin was dropped. A permeation ratio after 24 hours was obtained by measuring fluorescence of a back surface of the insert and by using an image analysis system (IVIS Lumina II, PerkinElmer Co., Ltd.). Fig. 8 shows the results.

As shown in Fig. 8, Example 1 showed significantly low protein permeability (about 20%) compared with those

of the fibrin film and the Seprafilm, which means that most protein remained on the surface. This suppressed protein permeability is considered to contribute to the adhesion prevention property. Although underivatized gelatin film also showed a low permeability, the protein spread over the entire thickness.

<Degree of Swelling>

The first agent and the second agent of each of Example 1 and Comparative Example 1 were poured each in an amount of 1000 μL between two glass plates sandwiching a 0.5 mm-thick silicone rubber spacer to generate a plate-shaped cured adhesion prevention material. A 0.5 mm-thick circular film having a diameter of 4 mm was punched out from the obtained cured adhesion prevention material. A weight ($W_d$) of the film after freeze-dried was measured. The film was dipped in saline at 37°C and a degree of swelling was calculated from a change in weight ($W_s$) of the adhesion prevention material film with time in accordance with the following equation.

$$\text{Degree of swelling (\%)} = \{(W_s - W_d)/W_d\} \times 100$$

Fig. 9 shows the change in degree of swelling with time. No significant difference was observed between both samples in the test period.

<Enzymatic Degradability>

The first agent and the second agent of each of Example 1 and Comparative Example 1 were poured each in an amount of 1000 μL between two glass plates sandwiching a 1.0 mm-thick silicone rubber spacer to generate a plate-shaped cured adhesion prevention material. A 1.0 mm-thick circular film having a diameter of 10 mm was punched out from the obtained cured adhesion prevention material of which initial weight ($W_{t0}$) was measured. To prevent formation of calcium salt with phosphate buffered saline (PBS), the specimen was soaked in a tris-hydrochloric acid buffer (2.5 mM, $CaCl_2$, pH 7.4) for one night. Collagenase was dissolved in the tris-hydrochloric acid buffer at a concentration of 100 μg/mL, and the specimen was dipped in 2 mL of the collagenase solution. The weight (%) of the specimen remaining without being decomposed was calculated from a change in weight (Wc) of the adhesion prevention material film with time according to the following equation.

$$\text{Weight (\%)} = (W_t/W_{t0}) \times 100$$

In the formula, $W_{t0}$ represents the initial weight and Wt represents a weight at a time t after the specimen was soaked. Fig. 10 shows the results. As shown in Fig. 10, the film of Example exhibited a decomposition time longer than that of Comparative Example and it was confirmed that the film is able to protect a surgical site for longer time.

## INDUSTRIAL APPLICABILITY

The adhesion prevention material for use as defined in the appended claims is applicable to a surgical site having a complicated shape, and is very suited for clinical use as an adhesion prevention material having adhesiveness or as a surgical sealant having adhesion prevention property. Further, the adhesion prevention method disclosed is applicable to a site difficult for conventional methods.

## Claims

**1.** Material for use in preventing a surgical site from adhering after surgery, said material consisting of:

(1) a first agent comprising a gelatin derivative, the gelatin derivative

(a) including a structure represented by the following formula,

$$GltnNH\text{-}CHR^1R^2,$$

in the formula, Gltn represents a gelatin residue, $R^1$ represents an alkyl group with 5 to 17 carbon atoms, and $R^2$ represents a hydrogen atom or an alkyl group with 5 to 17 carbon atoms;

(b) having imino group/ amino group (molar ratio) of 1/99 to 30/70; and
(c) having a weight average molecular weight of 10,000 to 50,000; and

(2) a second agent comprising a crosslinker for the gelatin derivative.

**2.** Material for use according to claim 1, wherein the gelatin is a gelatin derived from Alaska Pollock.

**3.** Material for use according to claim 1 or 2, wherein $R^1$ represents an alkyl group with 5 to 11 carbon atoms, and $R^2$ represents a hydrogen atom.

**4.** Material for use according to any one of claims 1 to 3, wherein the crosslinker is a polyethylene glycol ether polybasic acid ester having at least two activated ester groups.

**5.** Material for use according to any one of claims 1 to 4, wherein the adhesion prevention material is a tissue adhesiveness adhesion prevention material.

## Patentansprüche

**1.** Material zur Verwendung beim Verhindern einer Adhäsion einer Operationsstelle nach einer Operation, wobei das Material aus Folgendem besteht:

(1) einem ersten Mittel umfassend ein Gelatinederivat, wobei das Gelatinederivat

(a) eine Struktur umfasst, die durch die folgende Formel

$$GltnNH\text{-}CHR^1R^2$$

repräsentiert wird, wobei in der Formel Gltn einen Gelatinerest repräsentiert, $R^1$ eine Alkylgruppe mit 5 bis 17 Kohlenstoffatomen repräsentiert, und $R^2$ ein Wasserstoffatom oder eine Alkylgruppe mit 5 bis 17 Kohlenstoffatomen repräsentiert;
(b) Iminogruppen/Aminogruppen (molares Verhältnis) von 1/99 bis 30/70 aufweist; und
(c) ein mittleres Molekulargewicht von 10.000 bis 50.000 aufweist; und

(2) einem zweiten Mittel umfassend einen Vernetzer für das Gelatinederivat.

**2.** Material zur Verwendung nach Anspruch 1, wobei die Gelatine eine vom Alaska-Pollack stammende Gelatine ist.

**3.** Material zur Verwendung nach Anspruch 1 oder 2, wobei $R^1$ eine Alkylgruppe von 5 bis 11 Kohlenstoffatomen und $R^2$ ein Wasserstoffatom repräsentiert.

**4.** Material zur Verwendung nach einem der Ansprüche 1 bis 3, wobei der Vernetzer ein Ester von Polyethylenglykol mit einer mehrwertigen Säure ist, der mindestens zwei aktivierte Estergruppen aufweist.

**5.** Material zur Verwendung nach einem der Ansprüche 1 bis 4, wobei das eine Adhäsion verhindernde Material ein Material zum Verhindern einer Adhäsion durch Gewebeadhäsivität ist.

## Revendications

**1.** Matière pour utilisation dans la prévention de l'adhérence à un site chirurgical après chirurgie, ladite matière consistant en :

(1) un premier agent comprenant un dérivé de gélatine, le dérivé de gélatine

(a) incluant une structure représentée par la formule suivante,

$$GltnNH\text{-}CHR^1R^2,$$

dans la formule, Gltn représente un résidu de gélatine, $R^1$ représente un groupe alkyle avec 5 à 17 atomes de carbone et $R^2$ représente un atome d'hydrogène ou un groupe alkyle avec 5 à 17 atomes de carbone ;
(b) présentant un groupe imino/un groupe amino (rapport molaire) de 1/99 à 30/70 ; et
(c) présentant une masse moléculaire moyenne en poids de 10 000 à 50 000 ; et

(2) un second agent comprenant un agent de réticulation pour le dérivé de gélatine.

**2.** Matière pour utilisation selon la revendication 1, dans laquelle la gélatine est une gélatine dérivée du colin d'Alaska.

**3.** Matière pour utilisation selon la revendication 1 ou 2, dans laquelle $R^1$ représente un groupe alkyle avec 5 à 11 atomes de carbone, et $R^2$ représente un atome d'hydrogène.

**4.** Matière pour utilisation selon l'une quelconque des revendications 1 à 3, dans laquelle l'agent de réticulation est un ester d'acide polybasique d'éther de polyéthylène glycol présentant au moins deux groupes ester activés.

**5.** Matière pour utilisation selon l'une quelconque des revendications 1 à 4, dans laquelle la matière anti-adhérence est une matière anti-adhérence à adhésivité tissulaire.

Fig. 1

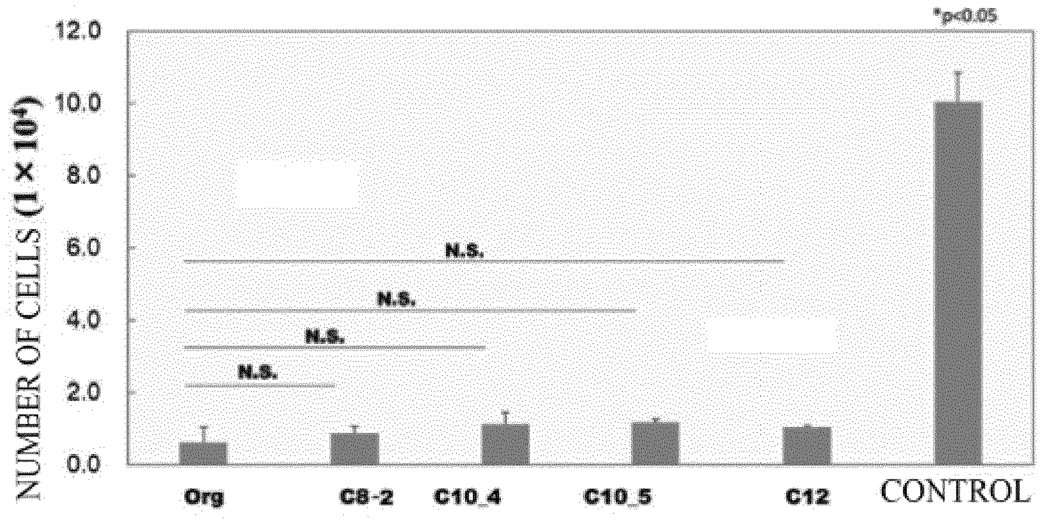
*p<0.05
12.0
10.0
8.0
6.0
4.0
2.0
0.0
NUMBER OF CELLS (1 × 10⁴)
N.S.
N.S.
N.S.
N.S.
Org
C8-2
C10_4
C10_5
C12
CONTROL

Fig. 2

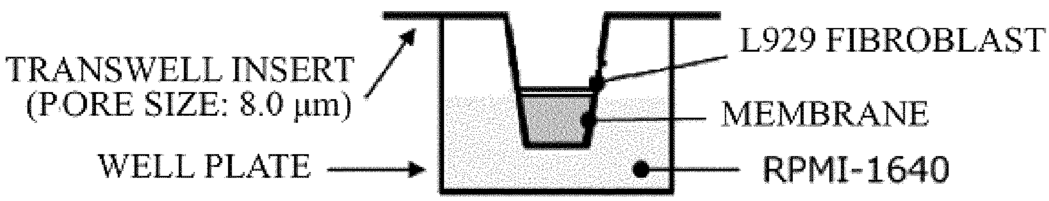
TRANSWELL INSERT
(PORE SIZE: 8.0 µm)
WELL PLATE
L929 FIBROBLAST
MEMBRANE
RPMI-1640

Fig. 3

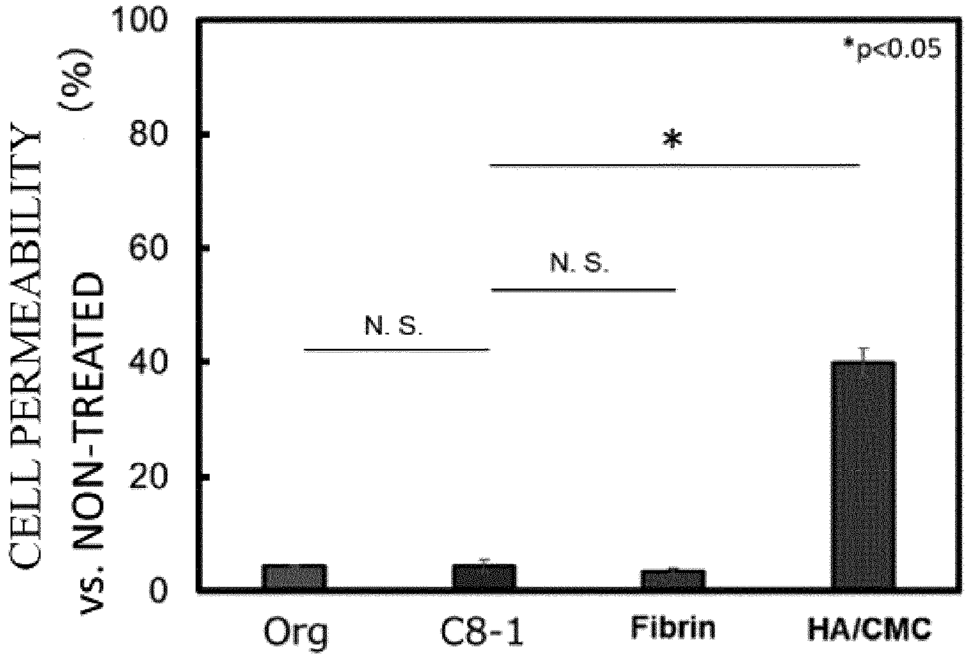
CELL PERMEABILITY
vs. NON-TREATED (%)
100
80
60
40
20
0
*p<0.05
*
N. S.
N. S.
Org
C8-1
Fibrin
HA/CMC

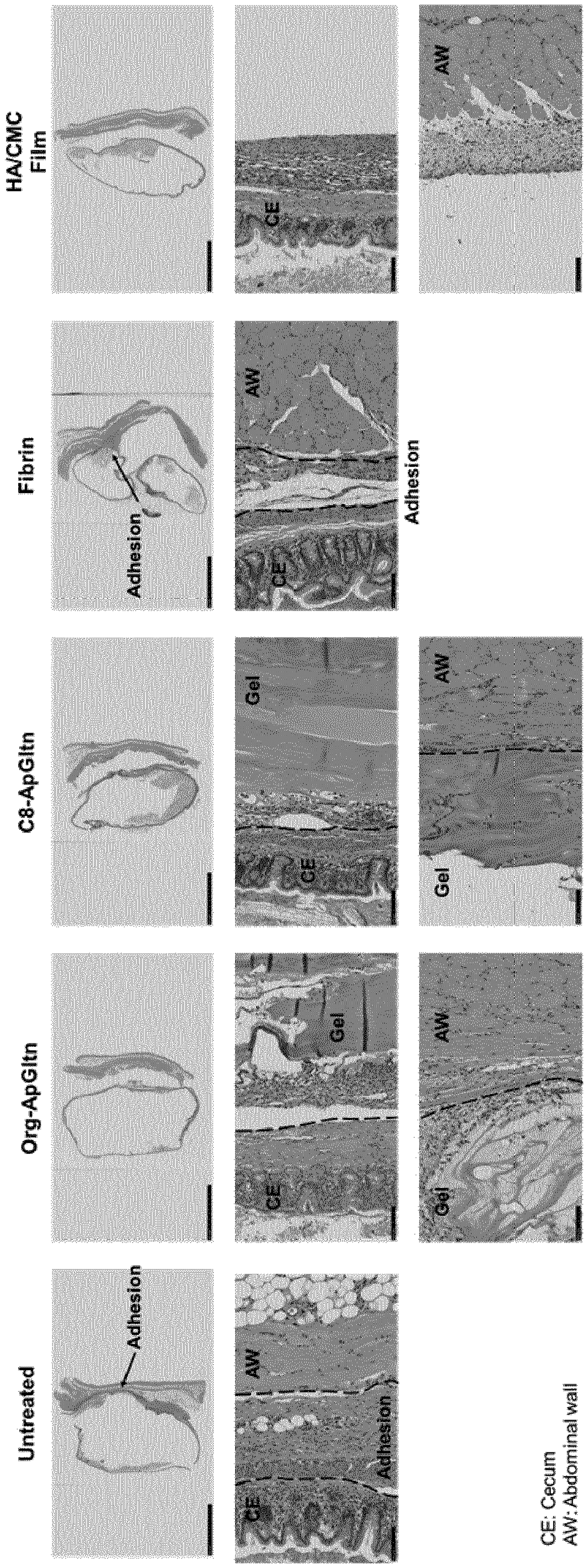
HA/CMC Film
Fibrin
C8-ApGltn
Org-ApGltn
Untreated
Adhesion
CE
AW
Gel
CE: Cecum
AW: Abdominal wall

Fig. 4a

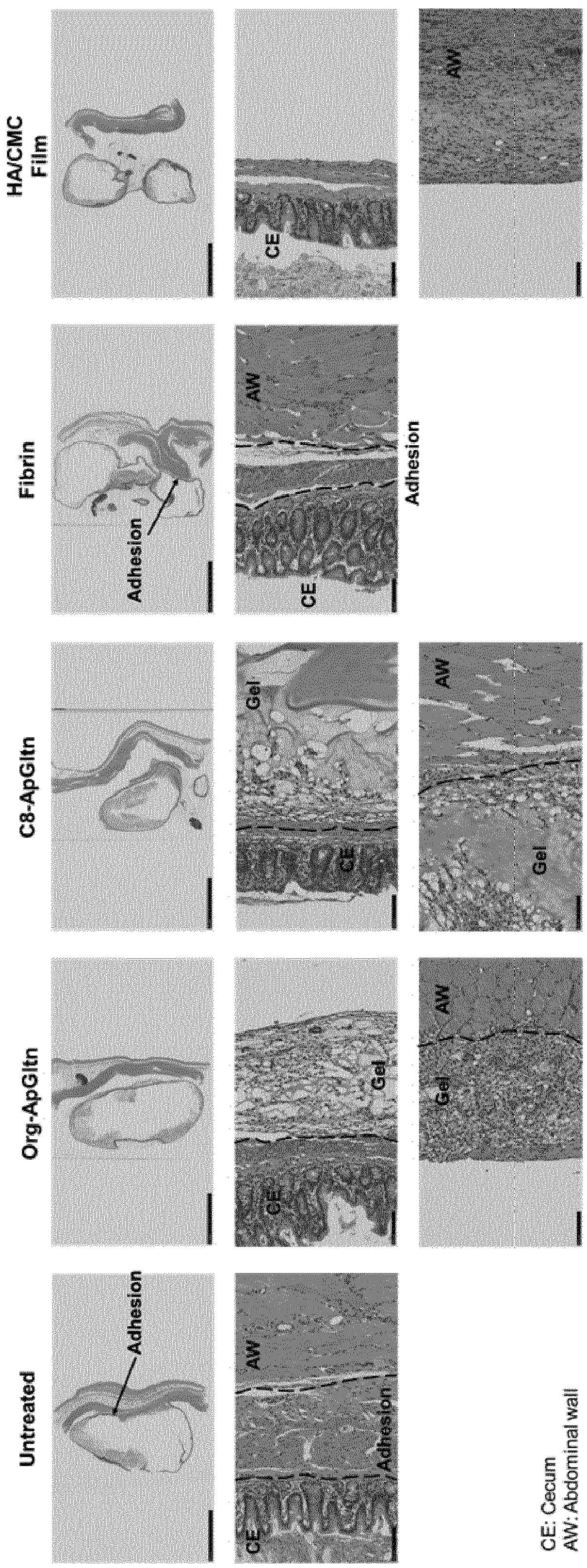
Untreated
Org-ApGltn
C8-ApGltn
Fibrin
HA/CMC Film
Adhesion
AW
CE
Gel
CE: Cecum
AW: Abdominal wall

Fig. 4b

Fig. 5

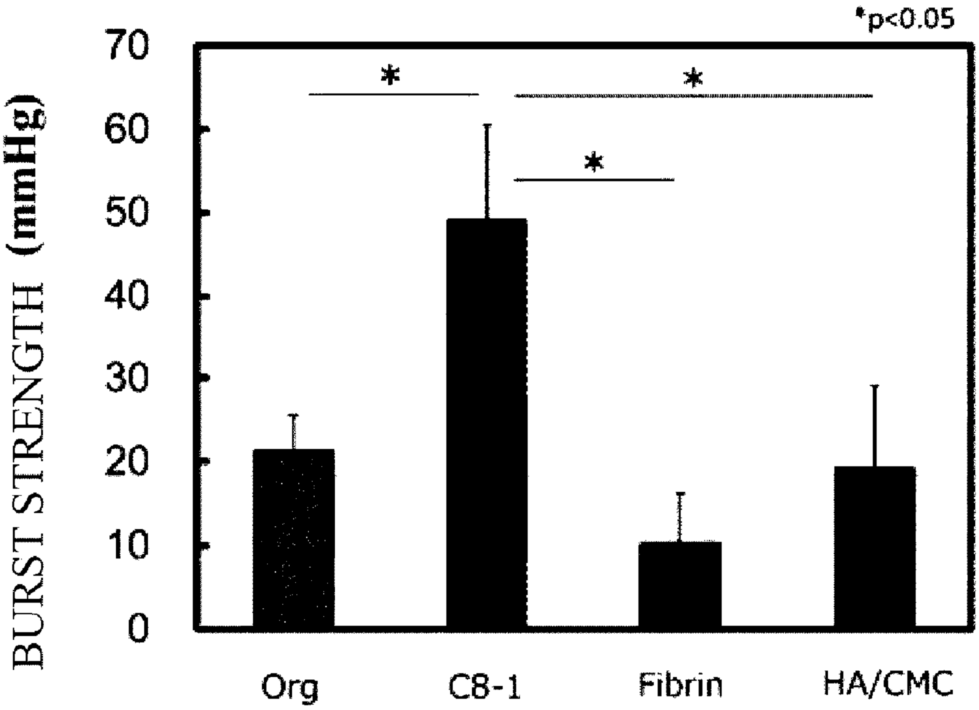
*p<0.05
70
60
50
40
30
20
10
0
BURST STRENGTH (mmHg)
*
*
*
Org
C8-1
Fibrin
HA/CMC

Fig. 6

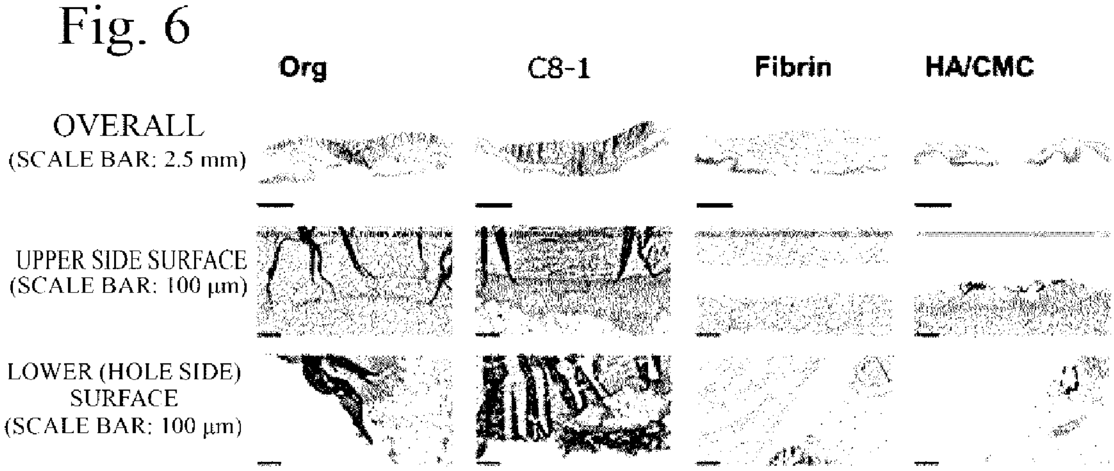
Org
C8-1
Fibrin
HA/CMC
OVERALL
(SCALE BAR: 2.5 mm)
UPPER SIDE SURFACE
(SCALE BAR: 100 μm)
LOWER (HOLE SIDE)
SURFACE
(SCALE BAR: 100 μm)

Fig. 7

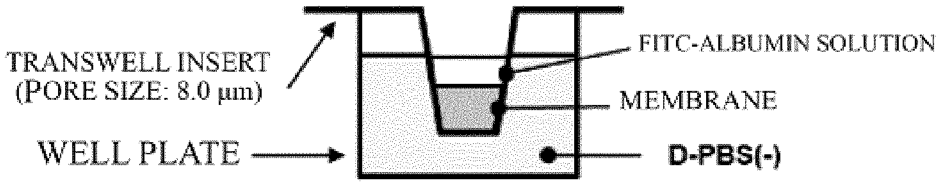
TRANSWELL INSERT
(PORE SIZE: 8.0 μm)
WELL PLATE
FITC-ALBUMIN SOLUTION
MEMBRANE
D-PBS(-)

Fig. 8

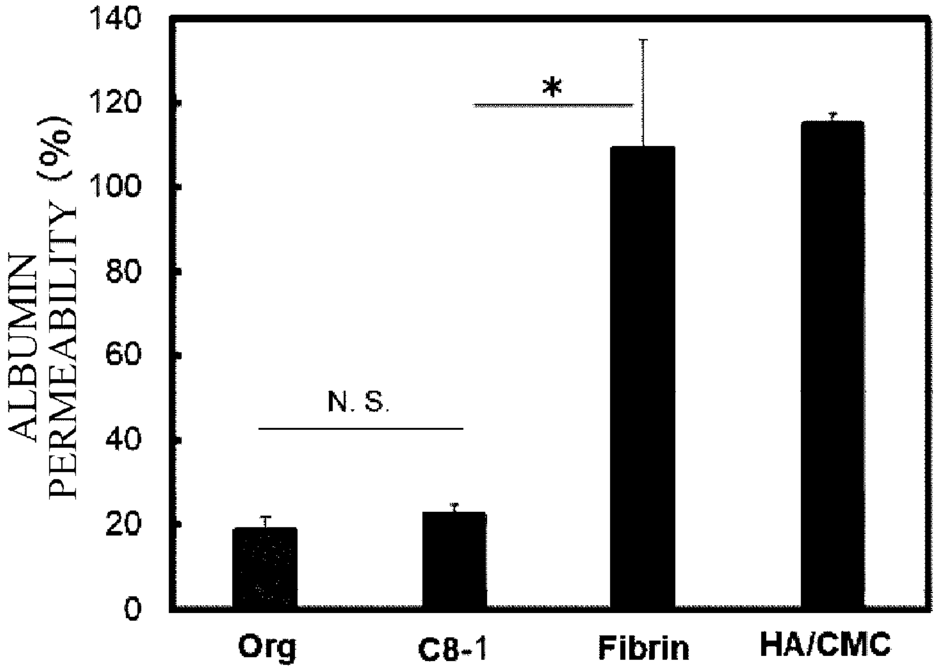
ALBUMIN PERMEABILITY (%)
140
120
100
80
60
40
20
0
*
N. S.
Org
C8-1
Fibrin
HA/CMC

Fig. 9

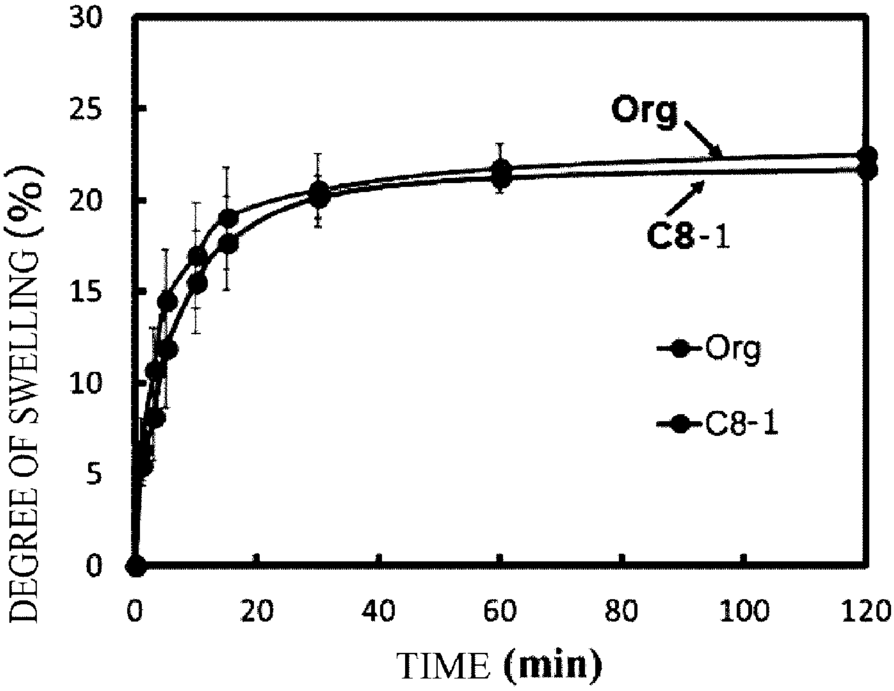
30
25
20
15
10
5
0
DEGREE OF SWELLING (%)
0
20
40
60
80
100
120
TIME (min)
Org
C8-1
Org
C8-1

Fig. 10

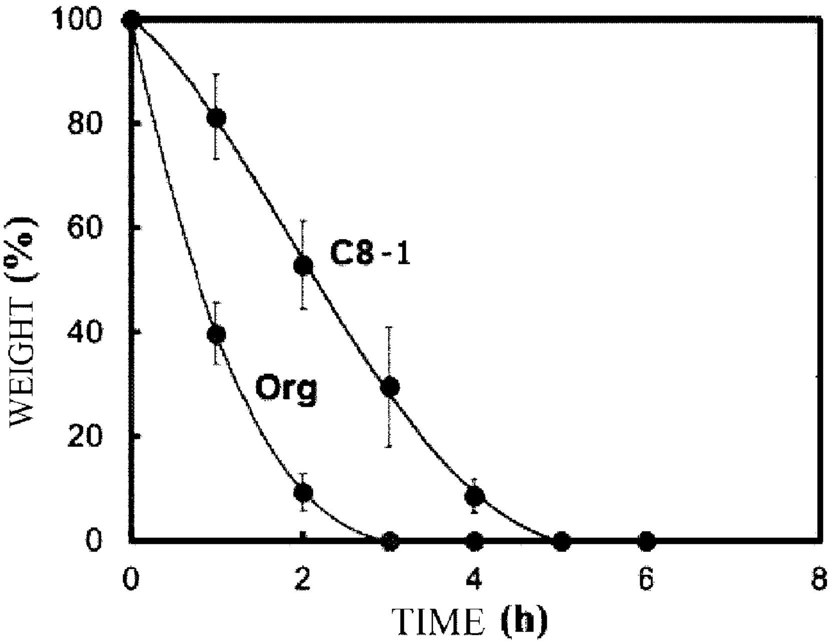
100
80
60
40
20
0
WEIGHT (%)
0
2
4
6
8
TIME (h)
C8-1
Org

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013226166 A
- WO 2014112208 A
- JP 5995128 B


### Non-patent literature cited in the description


- Specific Medical Treatment Materials and Their Material Prices (Material Price Reference). *Notification of the Ministry of Health, Labor and Welfare,* 2016, (402
- The Japanese Pharmacopoeia. 1235
- **D. NWA et al.** *J. Biomed. Mater. Res. B,* 2013, vol. 101B, 1251-1258